# EUROPEAN PATENT SPECIFICATION

(11) **EP 1 085 019 B1**
(45) Date of publication and mention of the grant of the patent: **29.01.2003**
(21) Application number: 00307729.4
(22) Date of filing: 07.09.2000
(51) Int. Cl.: C07D 401/12, A61K 31/4439, A61P 1/04

(54) **Omeprazole synthesis**
Verfahren zur Herstellung von Omeprazol
Procédé de préparation d'Oméprazole

(30) Priority: 13.09.1999 GB 9921578
(43) Date of publication of application: 21.03.2001
(73) Proprietor: Cipla Limited, Mumbai 400 008 (IN)
(72) Inventor: Hamied, Yusuf Khwaja, Dr., Windsor Villa, 2nd fl., Bombay 400 026 (IN); Kankan, Rajendra N, A-3/5, N BD Society, Mumbai 400 084, Maharashtra (IN); Rao,Dharmaraj R, 204 Shriji Krupa, Swaminarannager, Thane 400 601, Maharashtra (IN)
(74) Representative: Wain, Christopher Paul

(56) References cited:
- EP-A- 0 484 265
- US-A- 4 544 750
- PATENT ABSTRACTS OF JAPAN vol. 017, no. 391 (C-1087), 22 July 1993 (1993-07-22) & JP 05 070432 A (TOKYO TANABE CO LTD), 23 March 1993 (1993-03-23)

## Description

This invention relates to an improved method of making 5-methoxy-2-[[(4-methoxy-3,5-dimethyl-2-pyridinyl)-methyl) sulfinyl]-1H-benzimidazole, also known as omeprazole.

EP-B-0005129 describes certain substituted benzimidazole compounds among which is omeprazole. It has valuable properties in inhibiting gastric acid secretion. The patent describes a number of routes by which omeprazole may be prepared among which is the reaction of a compound of formula X with a compound of formula XI to form a compound of formula XII, which is then oxidised to omeprazole. 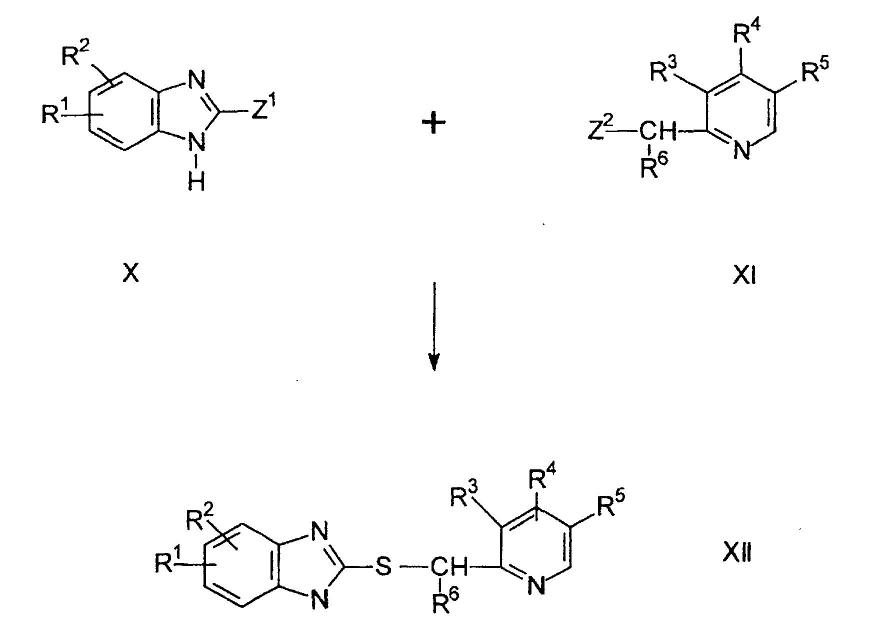 For the preparation of omeprazole, R¹ and R⁶ are hydrogen, R² and R⁴ are 5-methoxy, R³ and R⁵ are methyl, and Z¹ and Z² are each described as SH or a reactive esterified hydroxy group.

In GB-B-2126226 (and equivalent US 4544750), omeprazole is made by converting the group R in a compound of formula XIII to a CH₂Z group (Z is OH or a reactive ester group) and reducing the N-oxide to form a compound of formula XIV, which is then reacted with a benzimidazole: 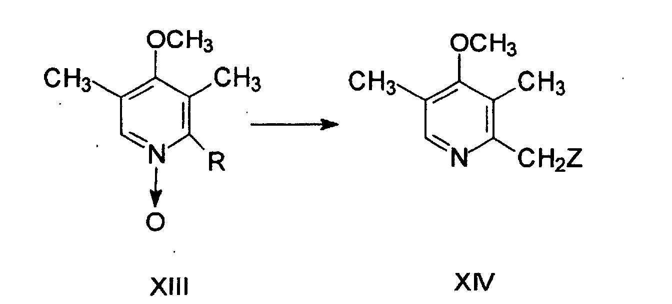 where R is H or CH₃.

JP 05070432 (Patent abstract) describes the production of the intermediate 2-hydroxymethyl-4-methoxy-3,5-dimethylpyridine (i.e. XIV above where Z = OH) from 4-methoxy-3,5-dimethyl-2-(-2-tertrahydropyramyloxymethyl) pyridine.

Whilst these processes are satisfactory, they have a number of drawbacks in terms of practicality, yield and general operation. We have now devised a new method of making omeprazole by which these drawbacks can be reduced or overcome.

In one aspect, the invention provides a process for the preparation of omeprazole which comprises the steps of:
(i) converting 4-nitro-2,3,5-trimethyl pyridine-N-oxide (I) to 4-nitro-3,5-dimethyl-2-(X-substituted methyl) pyridine (II) where X is halogen 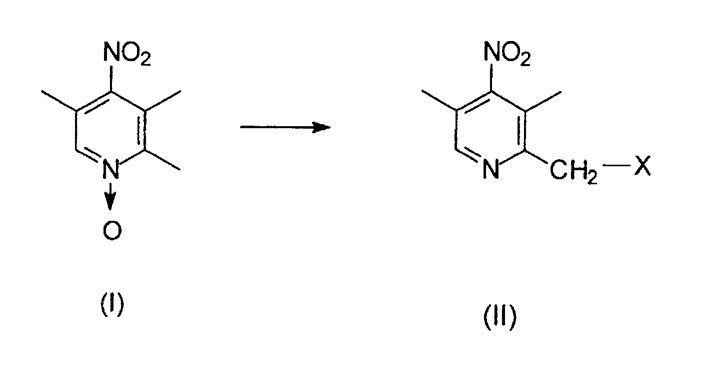
(ii)
   reacting (II) with a substituted benzimidazole of formula (III) to make a compound of formula (IV): 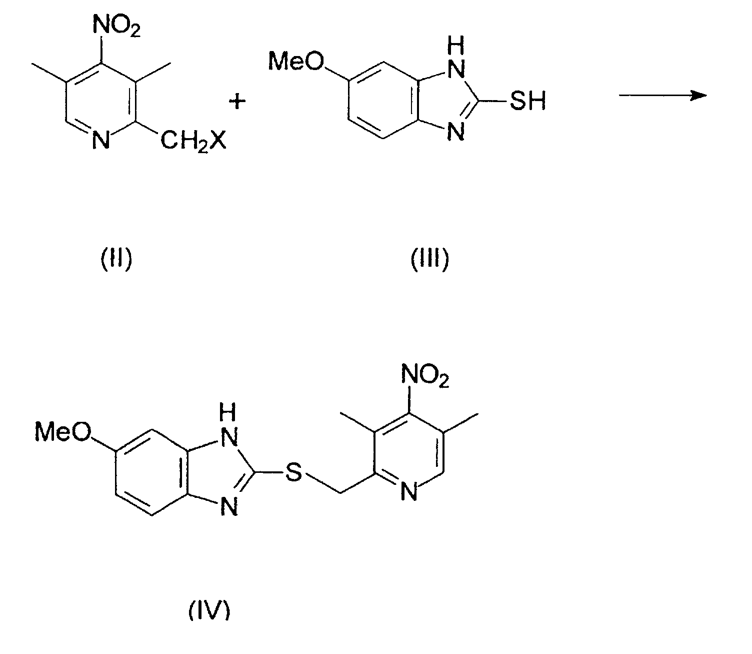
(iii) converting compound (IV) to compound (V) by replacing the nitro group with a methoxy group: 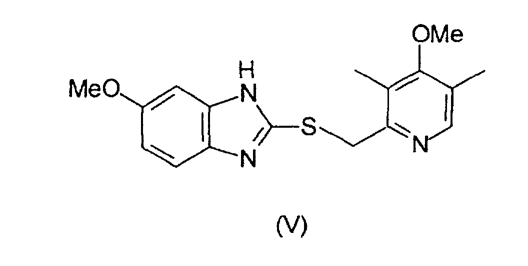
(iv) and oxidising (V) to form omeprazole (VI)

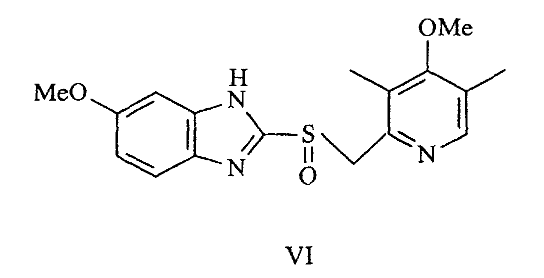

Preferably, in step (i), the compound I is acetylated to form 4-nitro-3,5-dimethyl-2-acetoxymethylpyridine (Ia) which, after hydrolysis to compound Ib, is halogenated to form the 2-halomethyl derivative (II): 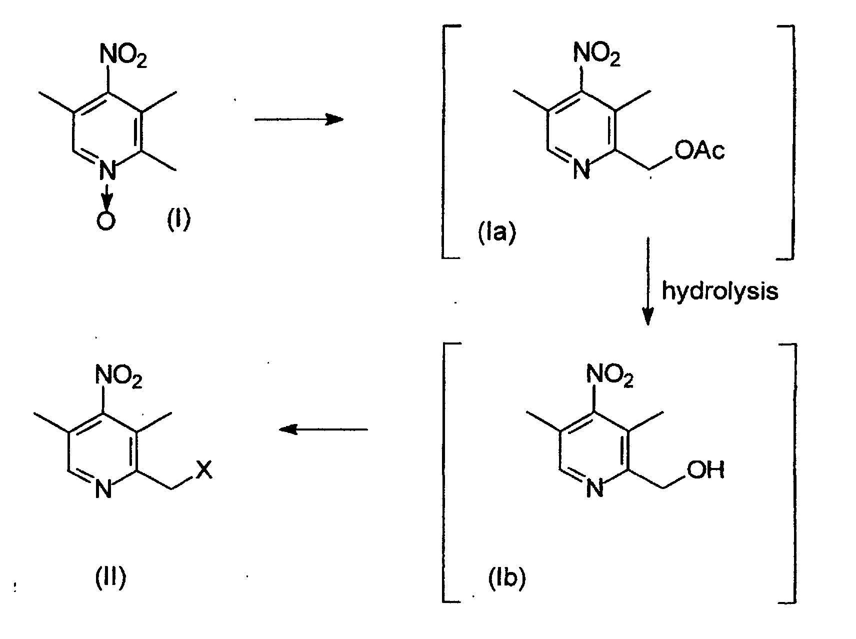 where X is halogen.

EP-A-484265 discloses a similar process, but with key differences. At page 32 of EP-A- 484265, production of omeprazole from the intermediate 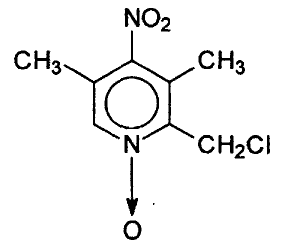 is described, but the N-oxide moiety is retained until after the condensation reaction with 5-methoxy-2-mercapto-benzimidazole has been performed, thus requiring a subsequent deoxygenation step.

In the above preferred procedure for step (i), the intermediate compounds Ia and Ib are preferably not isolated from the reaction mixture. Also, compound II need not be isolated.

In the most preferred step (i), the acetylation is effected using acetic anhydride, and the halogenation is effected using thionyl chloride. However, other suitable reactants can be used for each of these reactions.

Step (ii) of the process is preferably effected in the presence of sodium hydroxide and a phase transfer catalyst such as, for example, a quaternary ammonium salt, especially one selected from triethyl benzyl ammonium chloride, tetrabutyl ammonium bromide, tetrabutyl ammonium hydrogen sulphate and cetrimide.

Step (iii) of the process of the invention is preferably effected using a mixture of methanol, sodium methoxide and potassium carbonate. However, other reactants can be used such as, for example, potassium hydroxide, sodium hydroxide and potassium bicarbonate. In the most preferred method of performing step (iii), compound IV is reacted with potassium carbonate and sodium methoxide in methanol in the presence of catalytic amounts of anhydrous zinc chloride or magnesium chloride at a temperature above 45°C, preferably at the reflux temperature of methanol.

In step (iv) of the process, the compound of formula V is oxidised to form omeprazole. We prefer to use peracetic acid but other oxidants can be used, such as, for example, metachloroperbenzoic acid, magnesium monoperoxiphthalate and ammonium molybdate and hydrogen peroxide.

In order that the invention may be more fully understood, the following Examples are given by way of illustration only.

### Example 1

### Preparation of 4 -nitro-3,5-dimethyl-2-chloromethyl pyridine.hydrochloride.

In a reaction flask, acetic anhydride (1092 g; 10.7 mole) was heated to 60°C. To this was added under stirring, a solution of 4-nitro-2,3,5-trimethyl pyridine N-oxide (500 g; 2.74 mole) in acetic acid (650 ml). The contents were then heated to 110°C and maintained at this temperature for 30 minutes. Excess acetic anhydride and acetic acid (750 ml) were then distilled off under reduced pressure. To the cooled residue was added a 30% solution of sodium hydroxide to neutralise the remaining acids. This was then extracted with dichloromethane (2 x 1.2 lit). To the organic extracts was added 30% sodium hydroxide (1.20 lit) and the contents were heated to 45°C and maintained at this temperature for 4 hours. The organic layer was again separated and dried over anhydrous sodium sulphate. The dried organic extract was then transferred to a reaction flask. To it was slowly added thionyl chloride (580 g: 4.9 moles) at 10-15°C and the mixture stirred for 2 hours. The mixture was then quenched in ice-water, neutralised with 30% sodium hydroxide and extracted with dichloromethane (1.5 lit). The dichloromethane extract was dried over sodium sulphate. To this was then added 20% hydrochloric acid in isopropanol (350 ml) to crystallise the title compound which was filtered and dried. This afforded 475 g of (73%) of off-white crystalline product.

### Example 2

### Preparation of 5-methoxy-2-[((4-nitro-3,5-dimethyl-2-pyridinyl)methyl)-thio)-1H benzimidazole.

To a solution of the product from Example 1 (236 g; 1 mole) in water (750 ml) and dicholoromethane (500 ml) was added 5-methoxy-2-mercapto benzimidazole (180 g; 1 mole) and tetrabutyl ammonium bromide (5 g). To this was then added sodium hydroxide 30% solution (550 ml) and the mixture stirred at 25-30°C for 4 hours. The mixture was further diluted with water (1 lit) and the product was filtered and dried to obtain 312 g (91%) of the title compound as light yellow crystals.

### Example 3

### Preparation of 5-methoxy-2-[((4-methoxy-3,5-dimethyl-2-pyridinyl)methyl)-thio)-1H benzimidazole.

To a solution of the product from Example 2 (100 g; 0.29 mole) in methanol (500 ml), was added potassium carbonate (138g; 1.0 mole) followed by 30% sodium methoxide solution (330 ml; 2.0 moles) and anhydrous magnesium chloride (20 g). The contents were refluxed for 2 hours after which the reaction mixture was cooled to ambient temperature, filtered to remove inorganics and concentrated under reduced pressure. To the residue was added water (500 ml) and ethyl acetate (200 ml). The organic layer was separated, dried and chilled to 0.°C and kept overnight to afford 65g (75%) of the title compound as a crystalline powder.

### Example 4

### Preparation of Omeprazole

To a solution of 100 g of the product from Example 3 in ethyl acetate (500 ml) was added peracetic acid (125 g) slowly at a temperature of -25°C. The contents were further stirred for 30 minutes and the pH of the mixture was adjusted to 8 with 10% aqueous sodium hydroxide. The product thus precipitated was filtered and washed with water. The crude product was purified from methanol to afford 85g of pure omeprazole.

### Example 5

### Preparation of 5-methoxy-2-[((4-nitro-3,5-dimethyl-2-pyridinyl)methyl)thio)-1H benzimidazole.

In a reaction flask, acetic anhydride (109.2g: 1.07 mole) was heated to 50°C. To this was added under stirring, a solution of 4-nitro-2,3,5-trimethyl pyridine-N-oxide (50 g; 0.274 mole) in acetic acid (65 ml). The contents were then heated to 110°C and maintained at this temperature for 30 minutes. Excess acetic acid and acetic anhydride (75 ml) were then distilled off under reduced pressure. To the residue was added a 30% solution of sodium hydroxide to neutralise the remaining acid. Dichloromethane (120 ml) was then added to extract the product. To the organic extract was added 30% sodium hydroxide (120 ml) and the contents were heated to 45°C and maintained at this temperature for 4 hours. The organic layer was added slowly to thionyl chloride (58 g: 0.49 mole) at 10-15°C and the mixture stirred for 2 hours. The mass was quenched in ice-water (75 ml) and stirred for 30 minutes at 20-25°C. To this was added 5-methoxy-2-mercapto benzimidazole (32 g; 0.18 mole) and triethyl benzyl ammonium bromide (4 g) followed by 30% sodium hydroxide (110 ml). The reaction mass was stirred for 6 hours at 25-30°C after which it was diluted with water (300 ml) and filtered to obtain the title compound 65 g (69%).

## Claims

1. A process for the preparation of omeprazole which comprises the steps of:
(i) converting 4-nitro-2,3,5-trimethyl pyridine-N-oxide (I) to 4-nitro-3,5-dimethyl-2-(X-substituted methyl) pyridine (II) where X is halogen 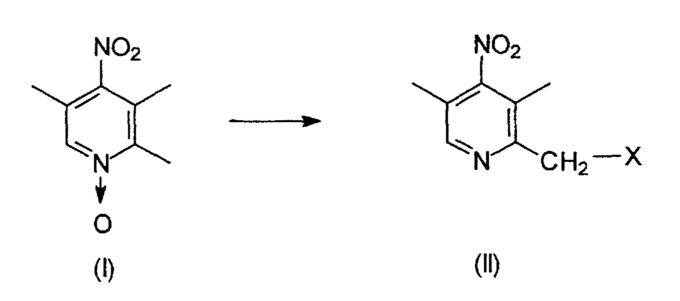
(ii) reacting (II) with a substituted benzimidazole of formula (III) to make a compound of formula (IV): 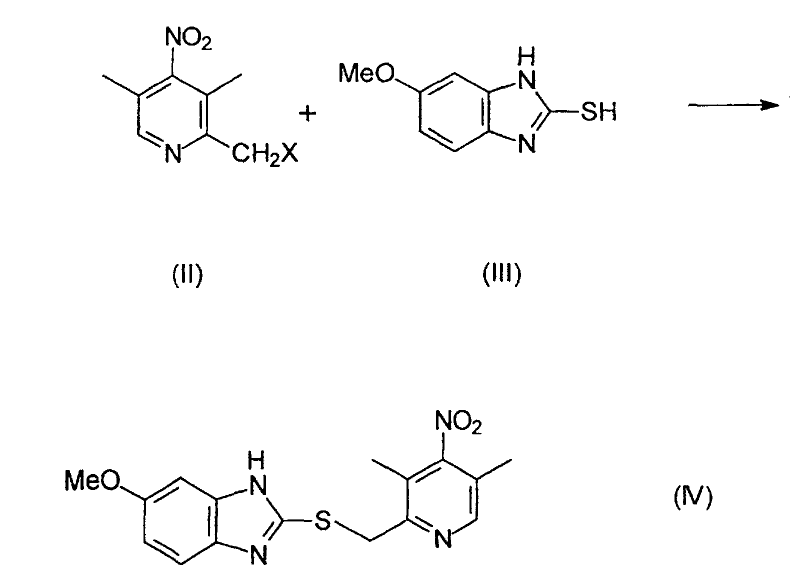
(iii) converting compound (IV) to compound (V) by replacing the nitro group with a methoxy group: 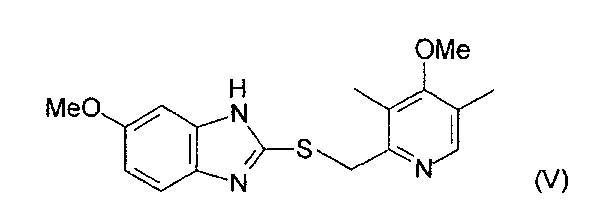
(iv) and oxidising (V) to form omeprazole (VI)

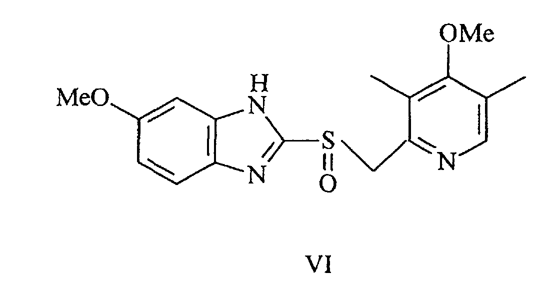

2. A process according to claim 1, wherein in step (i), the compound I is acetylated to form 4-nitro-3,5-dimethyl-2-acetoxymethyl pyridine (Ia) which, after hydrolysis to compound (Ib), is halogenated to form the 2-halomethyl derivative (II): 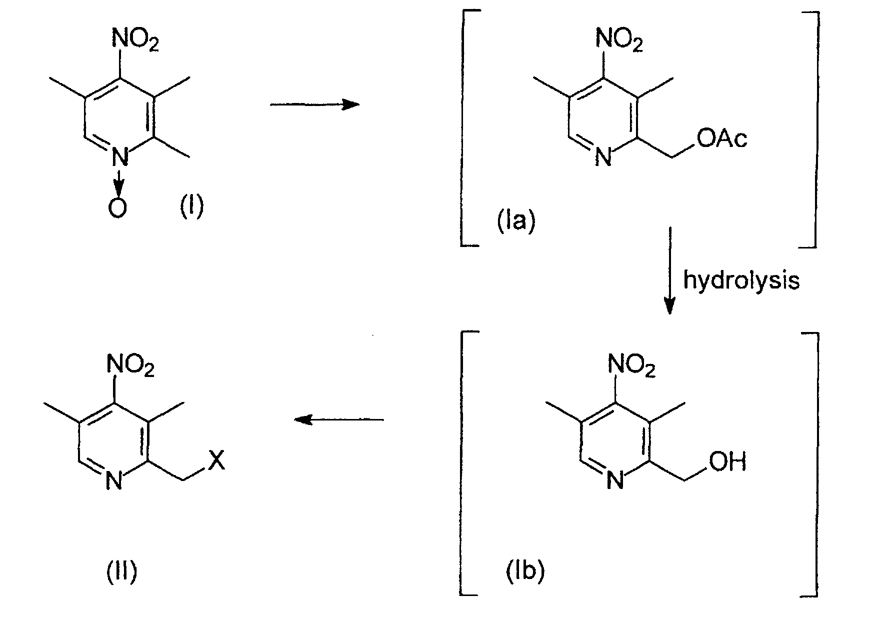 where X is halogen.

3. A process according to claim 2, wherein the acetylation is effected using acetic anhydride.

4. A process according to claim 2 or 3, wherein the halogenation is effected using thionyl chloride, the thionyl chloride preferably being in solution in methylene dichloride.

5. A process according to any of claims 1 to 4, wherein step (ii) is effected by reacting (II) with (III) in the presence of sodium hydroxide and a phase transfer catalyst.

6. A process according to claim 5, wherein the phase transfer catalyst is a quaternary ammonium salt selected from triethyl benzyl ammonium chloride, tetrabutyl ammonium bromide, tetrabutyl ammonium hydrogen sulphate and cetrimide.

7. A process according to any of claims 1 to 6, wherein step (iii) is effected by reacting (IV) with a methoxide in the presence of a base, preferably with a mixture of sodium methoxide and potassium carbonate in methanol.

8. A process according to claim 5, 6 or 7, wherein step (iii) is effected in the presence of a catalyst, preferably selected from anhydrous zinc chloride, anhydrous magnesium chloride and anhydrous calcium chloride.

9. A process according to any of claims 1 to 8, wherein step (iv) is effected using peracetic acid.

## Patentansprüche

1. Verfahren für die Zubereitung von Omeprazol, welches folgende Schritte umfasst:
(i) Umwandlung von 4-Nitro-2,3,5-trimethylpyridine-N-oxid (I) in 4-Nitro-3,5-dimethyl-2-(X-substituiertes Methyl)-Pyridin (II), wobei X für Halogen steht 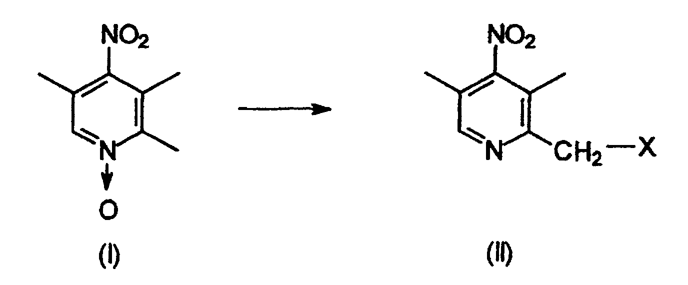
(ii) Reagieren von (II) mit einem substituierten Benzimidazol der Formel (III) unter Bildung einer Verbindung der Formel (IV): 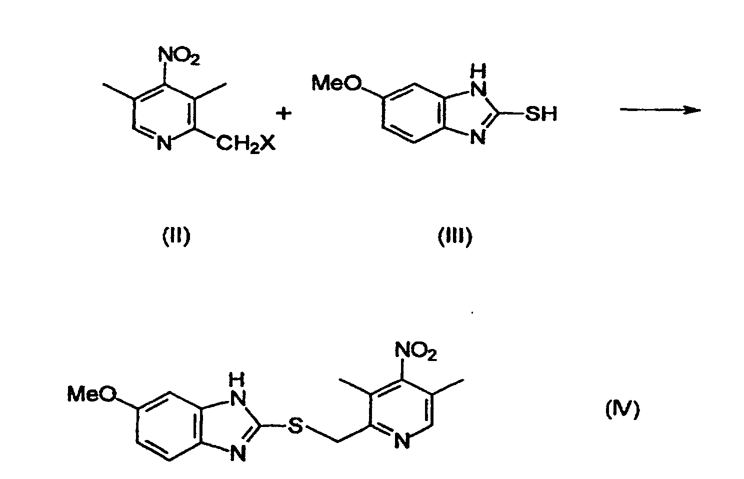
(iii) Umwandlung der Verbindung (IV) in Verbindung (V) durch Ersetzen der Nitro-Gruppe durch eine Methoxy-Gruppe: 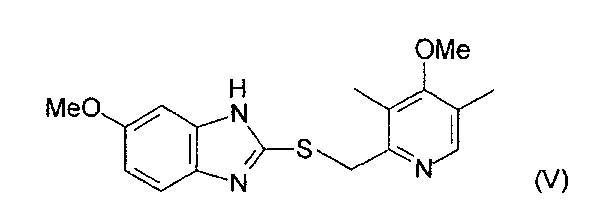
(iv) und Oxidieren von (V) unter Bildung von Omeprazol (VI)

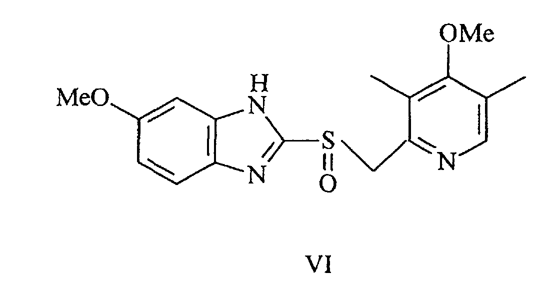

2. Verfahren nach Anspruch 1, wobei in Schritt (i) die Verbindung I acetyliert wird unter Bildung von 4-Nitro-3,5-dimethyl-2-acetoxymethylpyridin (Ia), das auf die Hydrolyse zur Verbindung (Ib) hin halogeniert wird unter Bildung des 2-Halomethyl-Derivats (II) : 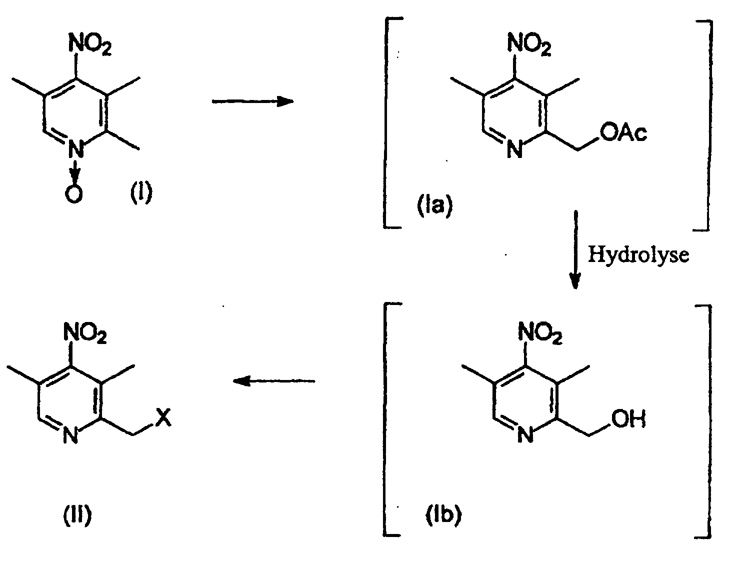 wobei X für Halogen steht.

3. Verfahren nach Anspruch 2, wobei die Acetylierung mit Hilfe von Essigsäureanhydrid durchgeführt wird.

4. Verfahren nach Anspruch 2 oder 3, wobei die Halogenierung mit Hilfe von Thionylchlorid durchgeführt wird, wobei sich das Thionylchlorid bevorzugt in Lösung in Methylendichlorid befindet.

5. Verfahren nach einem der Ansprüche 1 bis 4, wobei der Schritt (ii) durch Reagieren von (II) mit (III) in Gegenwart von Natriumhydroxid und einem Phasentransfer-Katalysator durchgeführt wird.

6. Verfahren nach Anspruch 5, wobei es sich bei dem Phasentransfer-Katalysator um ein quartäres Ammoniumsalz handelt, das unter Triethylbenzylammoniumchlorid, Tetrabutylammoniumbromid, Tetrabutylammoniumhydrogensulfat und Cetrimid durchgewählt wird.

7. Verfahren nach einem der Ansprüche 1 bis 6, wobei der Schritt (iii) durch Reagieren von (IV) mit einem Methoxid in Gegenwart einer Base, bevorzugt mit einer Mischung von Natriummethoxid und Kaliumcarbonat in Methanol durchgeführt wird.

8. Verfahren nach Anspruch 5, 6 oder 7, wobei der Schritt (iii) in Gegenwart eines Katalysators durchgeführt, der bevorzugt unter wasserfreiem Zinkchlorid, wasserfreiem Magnesiumchlorid und wasserfreiem Calciumchlorid durchgeführt wird.

9. Verfahren nach einem der Ansprüche 1 bis 8, wobei der Schritt (iv) mit Peressigsäure durchgeführt wird.

## Revendications

1. Un procédé pour la préparation d'oméprazole, qui comprend les étapes suivantes :
(i) conversion de 4-nitro-2,3,5-triméthylpyridine-N-oxyde(I) en 4-nitro-3,5-diméthyl-2(méthyle à substitution X)pyridine (II), X étant halogène 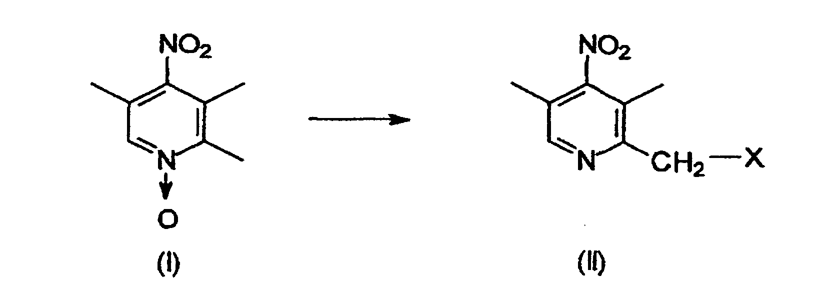
(ii) réaction de (II) avec un benzimidazole substitué ayant la formule (III) pour former un composé ayant la formule (IV) 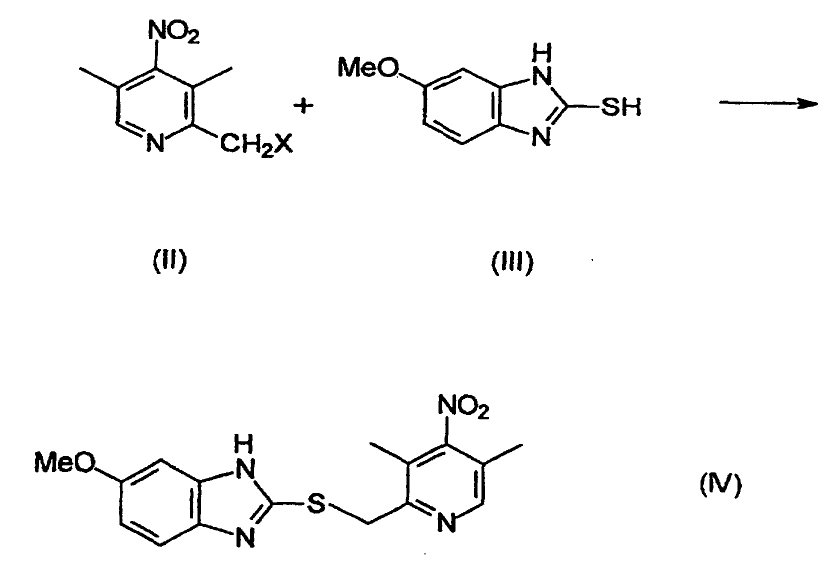
(iii) conversion du composé (IV) au composé (V) en remplaçant le groupe nitro par un groupe méthoxy : 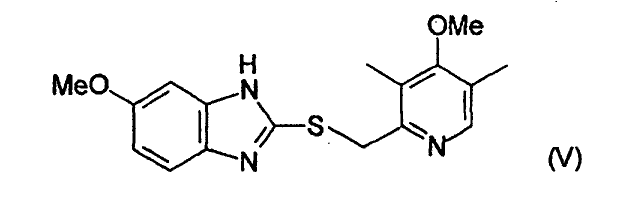
(iv) et oxydation de (V) pour former l'oméprazole (VI)

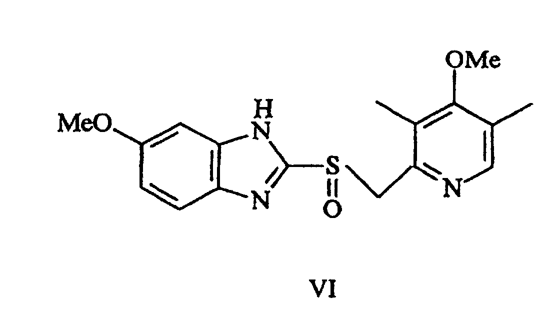

2. Un procédé selon la revendication 1, selon lequel, à l'étape (i), le composé I est acétylé pour former 4-nitro-3,5-diméthyl-2-acétoxyméthylpyridine(Ia) qui, après hydrolyse jusqu'à obtenir le composé (Ib), est halogénée pour former le dérivé 2-halométhyle (II). 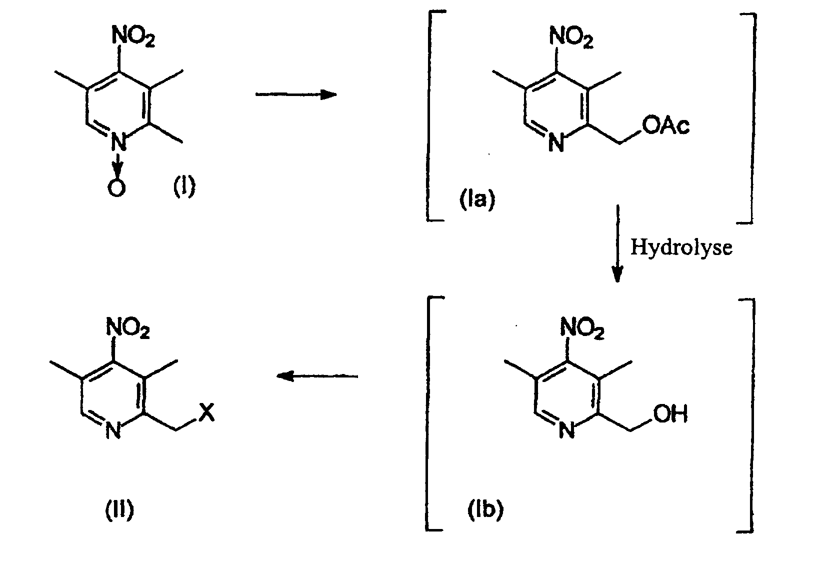 X étant halogène

3. Un procédé selon la revendication 2, selon lequel l'acétylation est réalisée en utilisant l'anhydride acétique.

4. Un procédé selon la revendication 2 ou 3, selon lequel l'halogénation est réalisée en utilisant le chlorure de thionyle, préférentiellement en solution dans du bichlorure de méthylène.

5. Un procédé selon l'une quelconque des revendications 1 à 4, selon lequel l'étape 5 est réalisée en faisant réagir (II) avec (III) en présence de soude caustique et d'un catalyseur à transfert de phase.

6. Un procédé selon la revendication 5, selon lequel le catalyseur à transfert de phase est un sel d'ammonium quaternaire sélectionné parmi : chlorure d'ammonium triéthylbenzylique, bromure d'ammonium tétrabutylique, hydrosulfate d'ammonium tétrabutylique et cétrimide.

7. Un procédé selon l'une quelconque des revendications 1 à 6, selon lequel l'étape (iii) est réalisée en faisant réagir (IV) avec un méthoxyde en présence d'une base, préférentiellement avec un mélange de méthoxyde de sodium et de carbonate de potassium dans du méthanol.

8. Un procédé selon la revendication 5, 6 ou 7, selon lequel l'étape (iii) est réalisée en présence d'un catalyseur, préférentiellement sélectionné parmi : chlorure de zinc anhydre, chlorure de magnésium anhydre et chlorure de calcium anhydre.

9. Un procédé selon l'une quelconque des revendications 1 à 8, selon lequel l'étape (iv) est réalisée en utilisant de l'acide peracétique.
